Europäisches Patentamt

European Patent Office

Office européen des brevets

(19)

(11) **EP 1 200 107 B1**

(12) **EUROPEAN PATENT SPECIFICATION**

(45) Date of publication and mention
of the grant of the patent:
**07.09.2005 Bulletin 2005/36**

(51) Int Cl.[7]: **A61K 35/78**, A61P 15/12

(86) International application number:
**PCT/DK2000/000406**

(21) Application number: **00945665.8**

(22) Date of filing: **17.07.2000**

(87) International publication number:
**WO 2001/005415 (25.01.2001 Gazette 2001/04)**

(54) **COMPOSITION HAVING STEROIDAL ESTROGEN EFFECT WITHOUT INCREASING THE RISK OF BREAST CANCER**

ZUSAMMENSETZUNG MIT STEORIDISHER ÖSTROGEN WIRKUNG OHNE STEIGERUNG DES BRUSTKREBSRISIKOS

COMPOSITION PRODUISANT UN EFFET OESTROGENE STEROIDE SANS AUGMENTATION DU RISQUE DE CANCER DU SEIN

(84) Designated Contracting States:
**AT BE CH CY DE DK ES FI FR GB GR IE IT LI LU MC NL PT SE**

(30) Priority: **15.07.1999 DK 103499**

(43) Date of publication of application:
**02.05.2002 Bulletin 2002/18**

(73) Proprietors:
• **Kobenhavns Universitet**
  **1070 Kobenhagen K (DK)**
• **Rigshospitalet**
  **2100 Copenhagen 0 (DK)**

(72) Inventors:
• **BRÜNNER, Nils**
  **DK-2900 Hellerup (DK)**
• **SPANG-THOMSEN, Mogens**
  **DK-2635 Ishoj (DK)**

(74) Representative: **Plougmann & Vingtoft A/S**
**Sundkrogsgade 9,**
**P.O. Box 831**
**2100 Copenhagen O (DK)**

(56) References cited:
**EP-A1- 0 847 755**

• **Fortschr. Med., Volume 114, No. 5, 1996, Von G. Rauthe, "Wechseljahresbeschwerden bei Mammakarzinompatientinnen" page 26; page 29-30, XP002901452,**
• **Journal of women's health. Mary Ann Liebert, Inc., Volume 7, No. 5, 1998, Shari Lieberman, Ph.D., C.N.S., "A Review of the Effectiveness of Cimicifuga racemosa (Black Cohosh) for the Symptoms of Menopause" page 525 - page 529, XP002901453**

**EP 1 200 107 B1**

**Description**

**Field of the invention**

The present application relates to the use of a composition comprising substances contained in Cimifuga Racemosa extract, for the preparation of a medicament for the treatment of estrogen deficiency. This composition has steroidal estrogen effect, however, does not have the steroidal estrogen side-effect of increasing the risk of development or the tendency to relapse of breast cancer.

**Background of the invention**

In women cessation of ovarian function is associated with various somatic and psychological disorders which are clinically summarised as menopausal symptoms. The most characteristic and frequent symptoms are cessation of menstrual bleeding, hot flushes, depression, nervousness and insomnia. In addition, many women will suffer from osteoporosis due to insufficient endogenous estrogen production. Menopausal symptoms are the result of substantially reduced steroid production of the ovaries. Thus, one possibility for treatment of menopausal symptoms is substitution treatment with ovarian hormones. Steroidal estrogens have been the treatment of choice, even though it is well known that this type of treatment may increase the risk of cancer development. In particular, for patients with a prior or a current breast cancer, it is advised not to use steriodal estrogens due the risk of stimulating dormant cancer cells.

An alternative to steroidal estrogens for the treatment of menopausal symptoms are plant estrogens (phytoestrogens). Among these, *Cimicifuga Racemosa* has been reported to be a successful therapeutic approach with beneficial effects on menopausal symptoms (1, 12-15, 20, 21). However, theoretically phytoestrogens would bind to estrogen receptors in target tissues including breast cancer cells in order to exert their estrogenic activity. It is therefore to be expected that phytoestrogens would stimulate growth of estrogen sensitive breast cancer cells. The study by Martin, P.M. et al. is in support of a growth stimulatory effect of plant estrogens on breast cancer cells as it is demonstrated herein that plant estrogens may interact with the estrogen receptors in human breast cancer cells in culture and thereby stimulate the growth of the cells (16). It would thus appear inadvisable to offer these compounds to women with a history of breast cancer or with a high risk of developing this disease.

*Cimicifuga Racemosa* extract and certain substances which are found, *inter alia*, in *Cimicifuga Racemosa* extract, have been suggested as estrogenic principles also in the patent literature, confer, e.g., US Patent No. 5,830,887, WO 98/50026 and EP 0847755.

A large fraction of women with early stage breast cancer (stage 1 and stage 2) experience cure of their disease after initial surgical treatment. It is, however, difficult to clearly define which women are most likely to have their disease return. Approximately 70% of all primary breast cancers express estrogen receptors and are therefore potentially estrogen sensitive and dependent, i.e. the growth of the cancer cells is stimulated by or dependent on estrogens (17, 18). It is therefore routinely recommended that these patients do not use steroidal estrogenic compounds at any time after the surgical resection of the primary breast cancer.

Currently available steroidal estrogens, which are most often used in hormone replacement therapy, do not discriminate between tissues or cell types. Thus, steroidal estrogens being used in clinical practice today may, while they provide a positive effect on estrogen deficiency symptoms and diseases, also carry the potential of enhancing cancer risk in women since they can stimulate cellular proliferation in several types of tissues, most notably breast tissue. Women with a family history of breast cancer and thereby with increased risk of developing breast cancer are therefore advised not to use steroidal estrogenic compounds.

Thus, there is at present an unmet need for substances or compositions with a differential estrogenic effect, i.e. compounds which by their estrogenic effects would relieve menopausal symptoms and other estrogen deficiency-related symptoms, but without stimulatory effect on the development and/or growth of breast cancer.

**Summary of the invention**

The present invention relates to means for relieving or curing symptoms or diseases which are caused by estrogen deficiency in mammals, in particular female mammals, in particular women, who suffer from breast cancer, or have a risk of recurrent breast cancer, or have a high risk of developing breast cancer.

Thus, in one aspect, the invention relates to the use of a composition comprising substances contained in Cimifuga Racemosa extract for preparing a medicament for use in a method for relieving or curing symptoms or diseases which are caused by estrogen deficiency, in a mammal who suffers from breast cancer, or has a risk of recurrent breast cancer, or has a high risk of developing breast cancer. The composition has an estrogen-like effect, as evidenced by a capability of the composition of inducing known physiological estrogenic effects in target tissues, and is free from interaction with breast cancer cells, in particular free from a stimulating effect on breast cancer thereby treating the estrogen deficiency-conditioned symptom or disease without introducing a risk of provoking the development of clinically evident breast cancer and/or stimulating growth of existing breast cancer cells in the mammal.

This means that treatment of the menopausal symptoms or other diseases based on estrogen deficiencies can now be performed without introducing the risk of stimulating breast cancer cells which is associated with administration of steroidal estrogen to a female mammal who suffers from breast cancer, or has a risk of recurrent breast cancer, or has a high risk of developing breast cancer.

The composition is a composition containing substances contained in *Cimicifuga Racemosa* extract. Thus, the composition may be or may contain *Cimicifuga Racemosa* extract (either in a liquid form or in the dry form). Doses of the extract may be from, e.g. 1 mg to 20 mg daily on the basis of the extract, such as 2-3 mg twice daily for a woman, and doses of other active principles than the *Cimicifuga Racemosa* extract described herein (SPP-001) can be assessed by the person skilled in the art based on the relative activity compared to SPP-001 extract, or based on the experiments explained in the following.

Another use of the invention is to combine the composition with various pharmaceutical compounds to broaden the estrogen like effect of these compounds without interfering with any simultaneous anti-estrogenic effects of the compounds on breast cancer cells. One example is raloxifene (methanone, [6-hydroxy-2-(4-hydroxyphenyl)benzo[b]thien-3-yl][4-(1-piperidinyl)ethoxy]phenyl]-hydrochloride) and associated compounds. Other examples are non-steroidal anti-estrogens such as tamoxifen, droloxifene. In addition the composition could also be combined with biphosphonates.

Based on the screening below substances or compositions contained in the Cimifuga Racemosa extract showing the valuable tissue-selective estrogenic effect may be identified.

The method for screening for substances or compositions contained in the Cimifuga Racemosa extract with the described properties comprises subjecting test substances or compositions to

1) testing for possible estrogen-like effect in normal tissue by measuring e.g. increase in uterine weight in an adult ovariectomized female rodent and

2) testing for possible estrogenic effect in breast cancer, and selecting, as candidates for tissue-selective estrogenic substances or compositions useful for relieving or curing symptoms or diseases which are caused by estrogen deficiency or which can be relieved or cured by administration of steriodal estrogen, in women, who suffer from breast cancer, or have a risk of recurrent breast cancer, or have a high risk of developing breast cancer, substances or compositions which,

a) are capable of inducing physiological estrogen effects e.g. increase in uterine wet weight or changes in gonadotropins or changes in bone mineral density, in an adult ovariectomized female rodent, and at the same time

b) have no effect on the growth of estrogen receptor-negative cancer cells and no effect on estrogen receptor-positive cancer cells in the doses in which they induce physiological estrogen effects, e.g. does not bind to the estrogen receptor in breast cancer cells.

This aspect of the invention is also explained in greater detail in the following, as are protocols for clinical work.

## DETAILED DESCRIPTION OF THE INVENTION

In Example 1 the estrogenic effect of SPP-001 on mouse uterine tissue is described. The dose selected was based on the recommended daily intake of SPP-001 in women, which is 2.4 mg/kg twice daily.

The increase in uterine weight induced by SPP-001 corresponds to that obtained following treatment with estradiol. Thus, it can be concluded that SPP-001 has estrogen-like effect on murine uterine tissue and that the applied administration and dose of SPP-001 is sufficient to induce this effect.

Example 2 describes the effect of SPP-001 on tumour development and growth of an estrogen receptor-negative human breast cancer grown in nude mice. Using the same dose of SPP-001 which induced increase in uterine weight, no effect was observed on the human breast cancer xenograft.

In Example 3, the estrogen receptor-positive human breast cancer cell line MCF-7 (ATCC HTB-22) was inoculated into nude mice and treated with estradiol or SPP-001. The SPP-001 dose was either the same as used in Example 1 and 2 or a 10 fold higher dose. The MCF-7 human breast cancer only forms tumours in ovariectomized or intact female nude mice in the presence of estrogen supplementation. In accordance with this, estrogen supplementation resulted in the development of growing tumours, while untreated mice did not develop any tumours. Treatment with SPP-001 in both dose groups did not result in tumour development in any of the mice studied. These results suggest that SPP-001 has no estrogenic effect on estrogen receptor-positive breast cancer cells.

Example 4 describes the effect of orally administered SPP-001 and subcutaneously administered estradiol on tumour development and tumour growth of the estrogen receptor-positive and estrogen dependent MCF-7 human breast cancer xenograft in nude mice. SPP-001 dose was 100 fold higher than the recommended daily dose for humans. The treatment with SPP-001 and estradiol was given both alone and in combination. SPP-001 had no growth supportive effect or growth inhibitory effect whether given alone or in combination with E2, suggesting that SPP-001 has neither potentiating nor inhibitory effect on estrogen sensitive breast cancer cells. The results further imply that SPP-001 does not bind to estrogen receptors of the MCF-7 cells, in that it does not antagonize the growth stimulatory effect of estradiol.

Example 5 describes an experimental model system for the testing of compositions or compounds ("drugs") with putative differential estrogen-like effects in normal tissues and in breast cancer. Possible estrogen-like effects in normal tissues are tested by e.g. measuring increase in mouse uterine weight as the end point, whereas possible estrogenic effect in breast cancer is tested by measuring growth supportive effect in the estrogen sensitive MCF-7 breast cancer xenograft as the end point. The tests can be performed in the same mice or in identical mice.

Example 6 describes a clinical protocol for safety studies of SPP-001 treatment in women with advanced breast cancer. Patients are randomized to treatment with SPP-001 or placebo and will be followed until death. Survival curves for each group will be constructed in order to determine the possible effect of SPP-001 in this patient population. It will be understood that this protocol can be adapted for use for safety studies of treatment with any CR-based composition which fulfils the criteria for being useful in the method according to the invention.

Example 7 describes a clinical protocol for safety studies of SPP-001 treatment in women with advanced breast cancer and who have obtained a complete or partial response upon conventional antineoplastic therapy. Patients are randomized to treatment with SPP-001 or placebo and will be followed until death. Time to progression in each treatment group will be determined and compared in order to establish whether SPP-001 has any effect on cancer progression in this patient population. Again, it will be understood that this protocol can be adapted for use for safety studies of treatment with any CR-based composition which fulfils the criteria for being useful in the method according to the invention.

In example 8, a clinical protocol is described for safety studies of SPP-001 treatment in women with a prior diagnosis of breast cancer and with no indication of recurrence. Patients are randomized to treatment with SPP-001 or placebo and will be followed for 5 years. Univariate recurrence-free and overall survival curves will be constructed and compared in order to establish whether SPP-001 has any effect on these two parameters. Also this protocol can be adapted for use for safety studies of treatment with any CR-based composition which fulfils the criteria for being useful in the method according to the invention.

Example 9 describes clinical SPP-001 treatment of estrogen deficiency conditions other than menopausal symptoms in women with current breast cancer, with a prior diagnosis of breast cancer, and with an increased risk of developing breast cancer. The diseases include osteoporosis, osteochondrosis, hyperlipidaemia, hypercholesterolaemia, arteriosclerosis and other conditions which can be cured or relieved by estrogen replacement therapy.

Thus a broad aspect of the present invention relates to the use of compositions or substances contained in Cimifuga Racemosa extract capable of inducing a physiological estrogen-like effect without interacting with breast cancer cells for the preparation of a medicament for the treatment of estrogen deficiency symptoms or diseases of a mammal suffering from or having a high risk of developing an estrogen dependent tumour.

The present invention is based on in vivo investigations performed on human breast cancer xenografts in immune deficient nude mice. Numerous studies have demonstrated that the responses of these breast cancer xenografts to endocrine treatment, i.e. ablative, inhibitive, additive; and competitive treatment are comparable to those obtained with these treatment modalities in breast cancer patients (2, 6-10). It is thus justified to presume that the response to SPP-001 of breast cancer *in situ* is comparable to the response obtained in the xenografts.

Estrogen has many physiological effects as estrogen is required for the normal maturation of the female e.g. by stimulating the development of the vagina, uterus, and uterine tubes as well as the secondary sex characteristics. Estrogen stimulates stromal development and ductal growth in the breast and is responsible for the accelerated growth phase and the closing of the epiphyses of the long bones that occur at puberty. Estrogen contributes to the growth of the axillary and pubic hair and alter the distribution of body fat so as to produce typical female body contours. Large quantities of estrogen also stimulate development of pigmentation in the skin most prominent in the region of the nipples and areolas and in the genital region. Estrogen also plays an important role in the development of the endometrial lining. Estrogen also haS a number of important metabolic effects. It is responsible for the maintenance of the normal structure of the skin and blood vessels in women. Estrogen decreases the rate of resorption of bone by antagonizing the effect of parathyroid hormone on bone but does not stimulate bone formation. In the liver, Estrogen alters the metabolism so that there is a higher circulating level of proteins such as transcortin (CBG), thyroxin binding globulin (TBG), sex hormone-binding globulin (SHBG), transferring, renin substrate, and fibrinogen. This leads to increased circulating levels of e.g. thyroxine, estrogen, testosterone, iron, and copper. Preferred physiological effects used as a measurement of consequence of treatment with the substance are stimulation of vaginal cells in, e.g., vaginal smear from a test animal, and measuring changes in gonadotropins, e.g. FSH and LH.

It will be obvious for the person skilled in the art to use one of the above mentioned physiological estrogen effects as a measure of estrogen-like effect for a substance. One of these physiological estrogen effects, the stimulation of uterine growth, have been chosen in the present technical teaching (examples 1-4). Thus, in a preferred embodiment of the invention the physiological estrogen-like effect is uterine growth as determined by an increase in uterine weight compared to controls after administration of the substance contained in Cimifuga Racemosa extract to ovariectomized female athymic nude mice. That is, a physiological estrogen-like effect is uterine growth as determined by an increase in mean uterine weight compared to controls of at least 0.10 g after administration of the substance to ovariectomized NMRI female athymic nude mice for 8 days.

The estrogen-like effect possessed by the composition used according to the invention manifests itself in the composition being capable of inducing an increase in uterine weight in adult ovariectomized NMRI female athymic nude mice, and for preferred compositions, the increase in uterine weight following a dose comparable to a normal dose for the mammal to be treated may correspond to a uterine weight increase seen in the same test animal following estradiol treatment, which will often be substantially the maximum uterine weight increase obtainable in the same test animal by estrogen treatment.

It is an important aspect of the invention that the substance contained in Cimifuga Racemosa extract is one which has substantially no effect on the growth of breast cancer cells, neither a positive, agonising, effect, nor a negative, antagonising, effect, and that this applies both for estrogen receptor-negative breast cancer cells (and also includes lack of indirect effect) and for estrogen receptor-positive breast cancer cells. The preferred lack of an antagonistic effect of the substance contained in Cimifuga Racemosa extract is based on the experimental reports demonstrating stimulation of breast and endometrial cancer cell growth following antiestrogen treatment of cells propagated in culture or athymic nude mice (22) and the often observed "flare" response to antiestrogens. It is preferred that the substance contained in Cimifuga Racemosa extract does not stimulate cancer cells that are estrogen receptor-positive. Likewise, it is preferred that said substance does not stimulate cancer cells that are estrogen receptor-negative. Furthermore, the substance should have no substantive effect on the breast cancer cells' capability of metastasizing or adhesion.

In one embodiment lack of stimulation of breast cancer cells is determined by no effect of the substance contained in Cimifuga Racemosa extract on growth of the estrogen and progesterone receptor negative MDA-MB-231 (ATCC HTB-26) human breast cancer cell line inoculated into six-week-old female athymic nude mice compared to a control wherein the tumours show increasing size during at least six consecutive growth recordings. Such method is also described in details in example 2.

In another embodiment lack of stimulation of breast cancer cells is determined by no effect of the substance contained in Cimifuga Racemosa extract on growth of the estrogen dependent and estrogen receptor-positive MCF-7 human breast cancer cell line inoculated into six-week-old female athymic nude mice compared to a control wherein the tumours show increasing size during at least six consecutive growth recordings. Such method is also described in details in example 3.

In a third embodiment lack of stimulation of breast cancer cells is determined by no effect of the substance contained in Cimifuga Racemosa extract when given in combination with estradiol on growth of the estrogen dependent and estrogen receptor-positive MCF-7 human breast cancer cell line inoculated into six-week-old female athymic nude mice compared to a control wherein the tumours show increasing size during at least six consecutive growth recordings. Such method is also described in details in example 4.

Ovariectomized rodents are suitable animal models for the study of estrogenic effects (2, 6-11). Administration of steroidal estrogens or other estrogenic compounds will result in increased uterine tissue weight. By using immune deficient rodents, the effects of steroidal estrogens and other estrogenic compounds can be studied simultaneously in the uterine tissue and in xenotransplanted human tumours.

The incidence of breast cancer is widespread. Women cured for breast cancer are still at risk of recurrent breast cancer, i.e. relapse. Quite a few women have a family history of breast cancer and are therefore at risk of developing breast cancer. Yet other women are in a position where they would prefer to minimise their discomfort during the menopause, without increasing their risk of breast cancer. Even though risk and high risk groups can be identified by statistical population studies, statistical grouping might not be relevant for the patient faced with the choice of minimising the discomfort during the menopause by traditional estrogen treatment versus the increased risk of breast cancer. The present substances will not put the female population in such unethical dilemma.

Thus based on the findings according to the invention, women who either suffer from breast cancer, or women who have suffered from breast cancer, and who in addition are suffering from menopausal symptoms, may due to the lack of estrogenic effects on breast cancer cells choose to use CR composition, e.g. SPP-001 to relieve their complaints. Likewise, women who have a high risk of developing breast cancer due to a family history of breast cancer and/or due to carcinoma *in situ* lesions in their breast tissue, may choose to use CR composition e.g. SPP-001 to relieve their menopausal symptoms.

Thus, based on the findings according to the invention, women who either suffer from breast cancer, or women who have suffered from breast cancer, and who in addition are suffering from menopausal symptoms, may due to the lack of estrogenic effects on breast cancer cells choose to use CR compositions, e.g. SPP-001, to relieve their complaints. Likewise, women who have a high risk of developing breast cancer due to a family history of breast cancer, genetic alterations related to an increase of breast cancer, nullipari, early menarche, late menopause, adipositas, prior use of steroidal estrogens, and carcinoma *in situ* lesion in their breast tissue, may choose to use CR composition, e. g. SPP-001 to relieve their menopausal symptoms.

In this context "high risk" is defined as a risk of developing breast cancer which is higher than 10% as compared to normal, i.e. a hazard ratio which is higher than 1,1 which is comparable to the increased risk of developing breast cancer when taking conventional contraceptive pills (hazard ratio of 1.1-1.2).

While by far the most important patient group to receive the treatment will be women, it is contemplated that the use of the invention may also be useful in human males, as it is also known that men may suffer from breast cancer and at the same time from symptoms or diseases which can be treated using estrogen.

As mentioned above, estrogen has a plethora of physiological effects. Thus, the symptoms of estrogen deficiency will vary. The substance contained in Cimifuga Racemosa extract will consequently alleviate estrogen deficiency-conditioned symptom or disease selected from the group consisting of menopausal symptoms; dermatological disorders such as ageing of the skin, wrinkles, dry skin and other estrogen deficiency related dermatological disorders; dryness of mucous membranes (e.g. vaginal and intestine); brain related disease such as Alzheimer's including other types of dementia; bone and joint related diseases such as osteoporosis, osteochondrosis, osteoarthritis, rheumatoid arthritis, healing of bone fractures, and reduction in skeletal fractures; vaginal estrogen deficiency such as vaginal dryness and dyspareuni; coronary heart diseases such as arteriosclerosis; and disease such as hyperlipidaemia and hypercholesterolaemia.

As the most common of these is menopausal symptoms, the treatment of these are preferred.

As illustrated in the examples, one substance contained in *Cimifuga Racemosa* extract that is capable of inducing a physiological estrogen-like effect without stimulating growth of breast cancer cells is *Cimicifuga Racemosa* extract (CR). Thus, a preferred substance to be used according to the invention is a composition containing substances contained in *Cimicifuga Racemosa* extract. Preferably a *Cimicifuga Racemosa* extract SPP-001.

Thus the invention is specifically based on the novel finding that a specific extract of CR (Cimicifuga racemosa) gives rise to a specific composition (SPP-001) which shows a unique combination of a very pronounced estrogenic effect, as assessed by support of uterus growth, and, in doses which give a maximum increase in uterus growth, comparable to the best increases obtainable by treatment with estradiol, shows a lack of estrogenic effects of SPP-001 on human breast cancer cells, hormone-sensitive and -dependent as well as hormone-resistant and -independent breast cancer cells. At present, it is not known whether this very beneficial combination is ascribable to one substance among the substances contained in the extract (some of which, such as formononetin, actein, 27-doxoactein, have been identified), but it seems likely that the effect is the effect of combinations of several substances in the extract, although it cannot be ruled out that it will be possible, using the principles for screening etc. which constitute an aspect of the present invention, to identify a single known or novel compound capable of exerting the valuable tissue-selective estrogenic effect.

In one aspect, the treatment according to the invention is based on extracts of *Cimicifuga Racemosa* (CR), which have known beneficial effects on menopausal problems in women (1, 12-15, 20, 21). Such extracts may be water soluble and/or substantially soluble in organic solvent systems, e.g., by alcohol extraction of *Cimicifuga Racemosa* material, in particular such as root stem. The estrogen-like effect of CR is further documented by the expected chances in gonadotropins following CR treatment (12, 14).

The composition may also be a composition comprising *Cimicifuga Racemosa* plant parts, or it may be a composition containing one or more chemical compounds contained in *Cimicifuga Racemosa* extract.

The composition may also be used in combination with a drug which has a selective estrogen receptor modulating (SERM) activity. Such drugs having SERM activity may be raloxifene (methanone, [6-hydroxy-2-(4-hydroxyphenyl)benzo[b]thien-3-yl][4-(1-piperidinyl)ethoxy]phenyl]-hydrochloride) and associated compounds. Other examples are non-steroidal anti-estrogens such as tamoxifen, droloxifene. In addition the composition could also be combined with biphosphonates.

The composition to be used according to the invention may be an oral composition or a composition suitable for any other appropriate route of administration, and examples of suitable pharmaceutical preparations can, e.g., be found in the literature, including the above-mentioned patent literature.

As indicated above, the findings according to the invention and the models developed by the inventors for assessing the suitability of a composition or a compound contained in Cimifuga Racemosa extract as a tissue-selective estrogenic substance open up the possibility of finding substances, compositions or chemical compounds which include the valuable tissue-selective estrogenic effect. Thus, a method for screening for substances or compositions contained in Cimifuga Racemosa extract which are capable of inducing a physiological estrogen-like effect without interacting with breast cancer cells is employed, said method comprising subjecting test substances or compositions to

1) testing for possible estrogen-like effect in normal tissue by measuring increase in uterine weight, changes in gonadotropins, changes in vaginal cytology and/or postmenopausal symptoms in an adult female mammal and 2) testing for possible estrogenic effect in breast cancer, and selecting, as candidates for tissue-selective estrogenic substances or compositions useful for relieving or curing symptoms or diseases which are caused by estrogen deficiency, or which can be relieved or cured by administration of steroidal estrogen, in a mammal who suffers from breast cancer, or has a risk of recurrent breast cancer, or has a high risk of developing breast cancer, substances or compositions which,

a) are capable of inducing physiological estrogenic effects in female mammals, and at the same time
b) have no effect on the growth of estrogen receptor-negative cancer cells and no effect on estrogen receptor-positive cancer cells in the doses in which they induce physiological estrogen effects.

It is preferred that the capability of the substance or composition contained in Cimifuga Racemosa extract of inducing physiological estrogen effects e.g. uterine growth female mammals is tested by testing the capability of the substance or composition of effecting uterine weight increase in ovariectomized female NMRI athymic nude mice, the lack of effect of the substance or composition on the growth of estrogen receptor-negative cancer cells is assessed as the lack of capability of the substance or composition of supporting growth of MDA-MB231 xenografts in female NMRI athymic nude mice, and the lack of effect of the substance or composition on the growth of estrogen receptor-positive cancer cells is assessed as the lack of capability of said substance or composition of supporting growth of MCF-7 xenografts in female NMRI athymic nude mice. Furthermore, said substance or compound should not support the growth of breast cancer cells even if combined with estradiol and even if the compound or substance is given in doses which are 10 or even 100 times higher than a dose giving, in the same strain of nude mice, a maximum uterus weight increase.

**Examples**

*Example 1: EFFECT OF ORALLYADMINISTERED SPP-001 ON UTERINE WEIGHT IN MICE*

This example describes the measurement of physiological estrogenic effects of orally administered SPP-001 in mice. As an example effect of orally administered SPP-001 on uterine weight in adult ovariectomized NMRI female athymic nude mice are given.

Ovariectomy, randomization and identification

After acclimatization the animals were ovariectomized and randomized to two treatment groups and a group of untreated controls (see below). The animals and cages were marked accordingly.

Study design

The mice (10 weeks old NMRI mice) were ovariectomized and randomized at day 0. One group of mice served as untreated controls (n=5), and one group of mice (n=5) was given estradiol from day 1 to 8 by insertion of a 0.72 mg subcutaneous E2 pellet. In the last group the mice (n=6) were given SPP-001 orally twice daily from day 1 to 8.

Observations

The animals were observed daily during the experimental period of 8 days.

CR extraction and dosing procedure

For extraction a standardised CR material as for example provided by Finzelberg AG, Germany (catalogue number 0472 312 or 0472 340) was used.
The desired extraction solution was made from 100 mg CR material and 1660 ml sterile water, vortexed and then kept at room temperature for 30 minutes. This final CR composition was named SPP-001. SP- 001 was made freshly for each experimental day and always stored at +4°C for a maximum of 24 hours. For each day of the experiment the above described procedure was repeated.
Dosing was performed orally with a dosing volume of 0.1 ml of SPP-001 to each mouse twice daily. Each mouse in the SPP-001 group received approximately 0.40-0.60 mg SPP-001 material/kg/day.

Estradiol (E2) treatment procedure

One day after ovariectomy, a 0.72 mg estradiol slow release pellet (Innovative Research, Toledo, USA, cat. no. SE-121) was inoculated subcutaneously in the neck of the mice using a trocar.

Termination of experiment

The experiment was terminated at day 8 after ovariectomy. All animals were sacrificed by cervical dislocation and the mouse uterine weights were determined.

Results

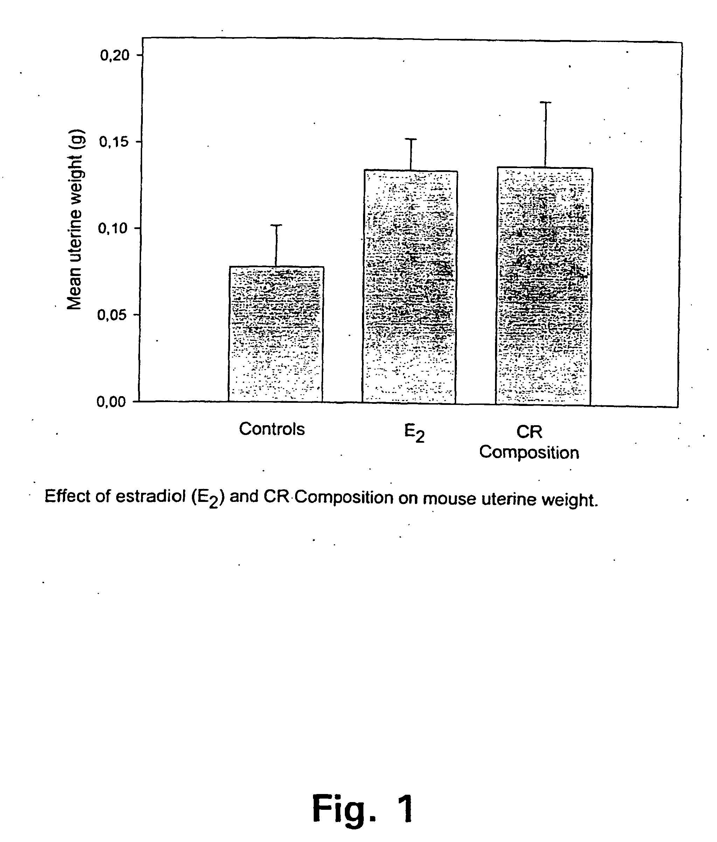

The calculated mean uterine weights and the corresponding ranges were 0.08 g [0.05 - 0.11], 0.13 g [0.11 - 0.15], and 0.14 g [0.11 - 0.21] in the control, estradiol and SPP-001 groups, respectively (*Table 1* and *Figure 1*).

Table 1

| Individual uterine weights (g) | | |
|---|---|---|
| Untreated controls | Estradiol (E$_2$) | CR composition |
| 0.09 | 0.11 | 0.14 |
| 0.11 | 0.15 | 0.11 |
| 0.08 | 0.14 | 0.13 |
| 0.06 | 0.12 | 0.21 |
| 0.05 | 0.15 | 0.11 |
|  |  | 0.12 |
| Effect of CR composition on uterine weight of ovariectomized adult NMRI/Bom™ female athymic nude mice. | | |

CR composition was given orally, 0.24 mg/kg twice daily. E$_2$ treatment was given by subcutaneous inoculation of a 0.72 mg slow release pellet (Innovative Research) in the neck.

Discussion

The SPP-001 dose was chosen to simulate the daily recommended dose.
SPP-001 had an *in vivo* effect on mouse uterine weight which was comparable to that seen following E2 treatment. Other methods to determine physiological estrogenic activity such as changes in gonadotropins (decrease) and changes in cytology of the vaginal cells (maintaining normal histological profile) can be used either in addition or as an alternative.

***Example 2: EFFECT OF ORALLY ADMINISTERED SPP-001 ON TUMOUR DEVELOPMENT AND GROWTH OF THE MDA-MB-231 HUMAN BREAST CANCER XENOGRAFT***

This example describes the effect of orally administered SPP-001 on tumour development and tumour growth of the estrogen receptor-negative and estrogen resistant and independent MDA-MB-231 human breast cancer xenograft in nude mice (4).

Cells

The human mammary cancer cell line MDA-MB-231 (ATCC HTB-26) was used. Near confluent *in vitro* grown cells were harvested using a cell scraper. After centrifugation an aliquot of the cells was stained by Trypan Blue and counted. The cells were resuspended in fresh medium to a final concentration of $1 \times 10^6$ viable cells per inoculum (0.2 ml).

Animals

The experiment was performed in a total of 21 six-week-old female META/Bom™ athymic nude mice. An acclimatization period of one week was allowed in order to exclude animals in poor condition.

The mice were kept under sterile conditions in laminar air flow clean benches. Type III Macrolon cages (42 x 26 x 15 cm) with five animals in each cage was used. The mice were allowed sterile water and food pellets *ad libitum.*
The cages and the bedding were changed once a week. The room temperature was $25 \pm 2°C$, and the relative humidity $55 \pm 5\%$. The room was illuminated 24 hours a day.

Animal randomization and identification

After acclimatization, the animals were randomized to a group of 11 treated animals and a group of 10 control mice. Each animal was identified by earmarks and each cage was marked to identify group and animal earmarks.

Study design

At day 0 the animals (n=21) were inoculated with tumour cells subcutaneously in both flanks.
The mice were then randomized into either treatment with SPP-001 (example 1) or treatment with sterile water. The mice of the SPP-001 treatment group were given SPP-001 orally (0,1 ml) twice daily from the day of cell inoculation and until termination of the experiment. Control mice received sterile water twice daily.

CR extraction and dosing procedure

As described in example 1.

Observations

The animals were observed daily during the experimental period of 38 days.
When the tumours became measurable they were measured in two dimensions three times a week using a sliding gauge. Tumour growth curves were constructed and growth curve parameters were calculated (19).
Tumours that did not grow during the entire experimental period and tumour inocula without take were excluded from the analysis.

Termination of experiment

The experiment was terminated when treated tumours had shown increasing size during at least six consecutive growth recordings. At termination of the experiment, the animals were sacrificed by cervical dislocation.

Results

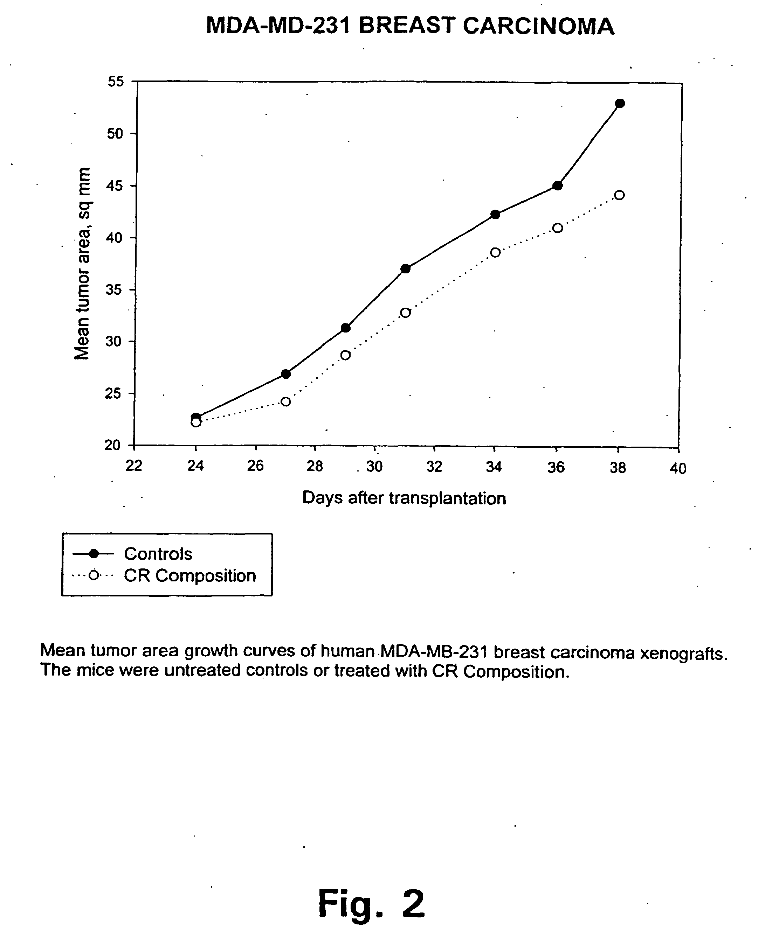

No visible signs of toxicity was seen in animals given SPP-001.
When this experiment was terminated, a total of 31 tumours in 17 mice were evaluable. 15 tumours developed in control mice and 16 tumours developed in the treated mice. *Figures 2* show the mean tumour area growth curves constructed from the individual tumour measurements. Calculated tumour growth curve parameters are listed in *Table 2*.
The specific growth rate, $\alpha$ (*Table 2*) of treated tumours was 0.0144 compared to 0.0165 of the vehicle treated control tumours, and as a consequence the calculated tumour volume doubling times (TD) were 14.9 days and 13.0 days of the treated and control tumours, respectively.

Table 2

| Group | Growth curve analysis | | | | |
|---|---|---|---|---|---|
| | Tumours[1] No. | Gompertz growth curve parameters[2] | | | $T_D$[3] days |
| | | $\alpha \times 10^3$ | $\beta \times 10^3$ | r | |
| Controls | 15 | 16.5 | 96.3 | 0.992 | 13.0 |
| CR composition | 16 | 14.4 | 80.3 | 0.994 | 14.9 |

Growth analysis of human MDA-MB-231 breast carcinoma xenografts grown in CR composition treated and untreated control female META/Bom™ athymic nude mice.
[1] Number of evaluable tumours; inocula without growth were excluded.
[2] The data on tumour area (product of two perpendicular measurements) were described according to a Gompertz function.

$$A(t) = A(0)\exp\{(1-\exp(-\alpha t))\beta/\alpha\}$$

A(0) is the tumour area at the time of tumour cell inoculation, A(t) is the tumour area at time t after inoculation, and $\alpha$ and $\beta$ are constants determining the course of the growth curves.

By linear regression the growth data were fitted to a straight lined transformation of the Gompertz function.

$$\ln[\ln A(max) - \ln A(t)] = \ln(\beta/\alpha) - \alpha t$$

A(max) is the theoretical maximal tumour area, $\alpha$ represents the slope, and r is the coefficient of correlation.

Tumour volume doubling time, $T_D$, was computed from the Gompertz parameters. Tumour volume was calculated from

$$V(t) = \Pi/6\ A^{3/2}\ k,$$

where A is tumour area, and k is a previously established constant of the relation between the two measurements obtained and the third dimension of the tumours.

Discussion

The results of the growth curve analysis indicate that the applied dose of SPP-001 had no effect on growth of the estrogen and progesterone receptor negative MDA-MB-231 human breast cancer cell line. Similar experiments as those described in this example can be performed using other estrogen receptor-negative human breast cancer cell lines, e.g. MDA-MB-435S (ATCC HTB 129).

**Example 3: EFFECT OF ORALLY ADMINISTERED SPP-001 ON TUMOUR DEVELOPMENT AND GROWTH OF THE MCF-7 HUMAN BREAST CANCER XENOGRAFT**

This example describes the effect of orally administered SPP-001 on tumour development and tumour growth of the estrogen receptor-positive and estrogen dependent MCF-7 human breast cancer xenograft in nude mice (4).

Cells

The estrogen and progesterone receptor positive human mammary cancer cell line MCF-7 (ATCC HTB-22) was used.

Near confluent *in vitro* grown cells were harvested using a cell scraper. After centrifugation an aliquot of the cells was stained by Trypan Blue and counted to determine the fraction of viable cells. The cells were resuspended in fresh medium to a final concentration of $10^6$ - $10^7$ viable cells per inoculum (0.1-0.2 ml). The cells were inoculated subcutaneously into both flanks of intact or ovariectomized female nude mice.

In separate experiments, xenotransplanted tumours in intact female nude mice were used as the source for the experiments. 1-2 mm diameter tumour blocks were prepared and inoculated into the right flank of recipient mice.

Animals

The experiment was performed in a total of 80 six-week-old female NMRI/Bom™ athymic nude mice. An acclimatisation period of one week was allowed in order to exclude animals in poor condition.

After acclimatisation some of the animals were ovariectomized under general anaesthesia and using standard procedures. Transplantation of tumours and inoculation of cells were performed following at least one week to ensure full recovery after the ovariectomy.

The mice were kept under sterile conditions in laminar air flow clean benches. Type III Macrolon cages (42 x 26 x 15 cm) with five animals in each cage were used. The mice were allowed sterile water and food pellets *ad libitum*.

The cages and the bedding were changed once a week. The room temperature was $25 \pm 2°C$, and the relative humidity $55 \pm 5\%$. The room was illuminated 24 hours a day.

Animal randomization and identification

After inoculation of cells or tumour blocks the animals were randomized into treatment groups according to the study design. Each animal was identified by earmarks and each cage was marked to identify group and animal earmarks.

Study design

Three series of experiments were performed.

*Study 1*   Forty ovariectomized animals were inoculated with $10^6$ MCF-7 cells, and the mice were randomized into four treatment groups: Untreated controls, 0.40-0.60 mg/kg/day SPP-001, 4 - 6 mg/kg/day SPP-001, and 0.72 mg estradiol slow release pellet (Innovative Research).

*Study 2*   Twenty intact female nude mice were inoculated with $10^7$ MCF-7 cells, and the mice were randomized into two treatment groups: 4 - 6 mg/kg/day SPP-001 or 0.72 mg estradiol slow release pellet (Innovative Research).

*Study 3*   Twenty intact female nude mice were inoculated with tumour blocks of MCF-7 xenografts into the right flank of recipient mice, and the mice were randomized into two treatment groups: 4 - 6 mg/kg/day SPP-001 or 0.72 mg estradiol slow release pellet (Innovative Research), respectively.

CR extraction and dosing procedure

Study 1. 0,1 ml twice daily of SPP-001 (as given in example 1) was given.
Study 2-3. 0,1 ml twice daily of a concentrated SPP-001 (1/10 volume water was used as described in example 1) was given.

Estradiol (E2) treatment procedure

At the day of inoculation of cells or tumour blocks a 0.72 mg estradiol slow release pellet (Innovative Research) was inoculated subcutaneously in the neck of the mice using a trocar.

Observation & calculations

The animals were observed daily during the experimental periods. When the tumours became measurable, they were measured in two dimensions three times a week using a sliding gauge. Tumour growth curves were constructed and growth curve parameters were calculated (19).

Termination of experiment

The experiment was terminated when growing tumours had shown increase in size during at least six consecutive growth recordings. At termination of the experiment, the animals were sacrificed by cervical dislocation.

Results

In non of the three studies toxicity was observed.

*Study 1*. The experiment was terminated 27 days after initiation because of a very low take rate. No tumours developed in the group of untreated controls or in the two treatment groups. However, only in one of the 10 E2 treated mice a growing tumour appeared.

*Study 2.* In this experiment six out of 9 evaluable E2 treated mice developed a total of 10 growing MCF-7 tumours. In contrast, in 10 out of 10 evaluable SPP-001 treated animals no tumours developed.

*Study 3.* When this experiment was terminated 52 days after transplantation, tumours had developed in 7 out of 10 mice in the E2 treatment group. No tumours were detected in 10 mice treated with the SPP-001.

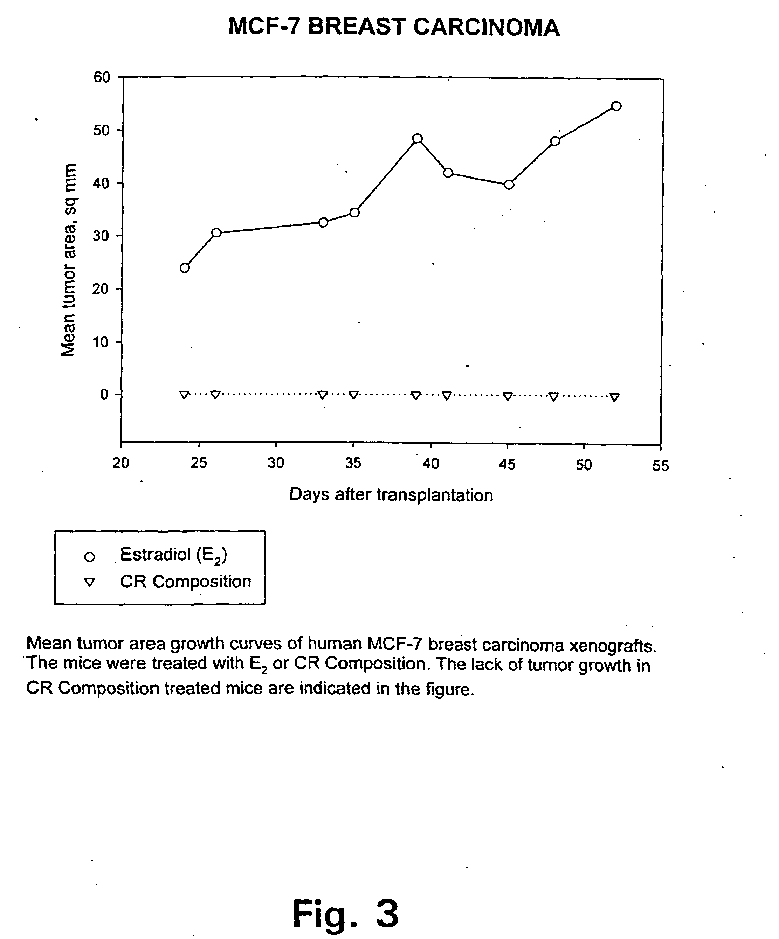

*Figure 3* shows the mean tumour area growth curve constructed from the individual tumour measurements of the E2 treated tumours, and the lack of tumour growth in SPP-001 treated mice is indicated in the figure. Calculated tumour growth curve parameters are listed in *Table 3.*

Table 3

| Group | Growth curve analysis | | | | |
|---|---|---|---|---|---|
| | Tumours No. | Gompertz growth curve parameters | | | $T_D$ days |
| | | $\alpha \times 10^3$ | $\beta \times 10^3$ | r | |
| Estradiol Estradiol ($E_2$) | 7/10 | 7.6 | 34.6 | 0.903 | 28.3 |
| CR composition | 0/10 | - | - | - | - |
| Growth analysis of human MCF-7 breast carcinoma xenografts grown in CR composition and $E_2$ treated female NMRI/Bom™ athymic nude mice. | | | | | |

Discussion

The presented series of experiments showed that the applied SPP-001 did not have tumour stimulatory effect on estrogen dependent MCF-7 human breast carcinoma xenografts. In contrast , treatment with E2 supported the growth of this tumour, thus confirming the ability of MCF-7 to form tumours in nude mice under adequate hormonal substitution. Similar experiments as those described in this example can be performed using other estrogen receptor-positive human breast cancer cell lines, e.g. T47D and ZR75-1 (5).

The results of *Study 2* and 3 were identical in the observed tumour growth despite the difference in method of tumour establishment, i.e. *in vitro* grown single cell suspensions vs. solid tumour blocks.

Although tumour growth was only observed in the E2 treatment group of *Study 1*, the results of this experiment was inconclusive due to the low take rate. This was probably caused by a relatively low cell dose of the inocula ($10^6$ cells). Therefore the cell dose was increased to $10^7$ cells/inoculum in *Study 2,* resulting in a take rate comparable to that obtained with transplantation of tumour blocks in *Study 3.*

The SPP-001 dose of 0.40-0.60 mg/kg/day was chosen to simulate the recommended daily intake of CR. The results showed that even a 10-fold increase in the dose of SPP-001 did not support the growth of MCF-7.

The MCF-7 breast carcinoma in nude mice is a slow growing tumour (3). The specific growth rate, $\alpha$ = 0.0076 (*Table 3*) corresponds to a tumour volume doubling time (TD) of 28.3 days.

*Example 4: EFFECT OF CONCOMITANT ORAL SPP-001 AND SUBCUTANEOUS ESTROGEN TREATMENT ON TUMOUR DEVELOPMENT AND TUMOUR GROWTH*

This example describes the effect of orally administered SPP-001 and subcutaneously administered estrogen on tumour development and tumour growth of the estrogen receptor-positive and estrogen dependent MCF-7 (ATCC HTB-22) human breast cancer xenograft in nude mice (4).

Cells

The estrogen and progesterone receptor positive human mammary cancer cell line MCF-7 was used.

Near confluent *in vitro* grown cells were harvested using a cell scraper. After centrifugation an aliquot of the cells was stained by Trypan Blue and counted to determine the fraction of viable cells. The cells were resuspended in fresh medium to a final concentration of $10^6$ - $10^7$ viable cells per inoculum (0.1-0.2 ml). The cells were inoculated subcutaneously into both flanks of intact female nude mice. One resulting MCF-7 tumour was used for serial transplantation into all the mice of this study.

Animals

The experiment was performed in a total of 32 six-week-old female NMRI/Bom™ athymic nude mice. An acclimatization period of one week was allowed in order to exclude animals in poor condition.

The mice were kept under sterile conditions in laminar air flow clean benches. The mice were allowed sterile water and food pellets *ad libitum.* The cages and the bedding were changed once a week. The room temperature was $25 \pm 2°C$. and the relative humidity $55 \pm 5\%$.

Animal randomization and identification

After inoculation of tumour blocks the animals were randomized into treatment groups according to the study design. Each animal was identified by earmarks and each cage was marked to identify group and animal earmarks.

Study design

Thirty-two intact female nude mice were transplanted with approximately 1-mm-diameter blocks of MCF-7 tumour into both flanks of recipient mice, and the mice were randomized into four treatment groups:

1:   40-60 mg/kg/day SPP-001 orally.
2:   0.72 mg slow release estradiol pellet s.c. (Innovative Research).
3:   40-60 mg/kg/day SPP-001 and 0.72 mg estradiol slow release pellet (Innovative Research).
4:   Sterile water p.o. twice daily.

CR extraction and dosing procedure

As described in example 1 except that a 100X dose was used.

Estradiol (E2) treatment procedure

At the day of inoculation of tumour blocks a 0.72 mg estradiol slow release pellet (Innovative Research) was inoculated subcutaneously in the neck of the mice using a trocar.

Observation & calculations

The animals were observed daily during the experimental period. When the tumours became measurable, they were measured in two dimensions three times a week using a sliding gauge. Tumour growth curves were constructed and growth curve parameters were calculated (19).

Termination of experiment

The experiment was terminated when the growing had shown increase in size during at least six consecutive growth recordings. At termination of the experiment, the animals were sacrificed by cervical dislocation.

Results

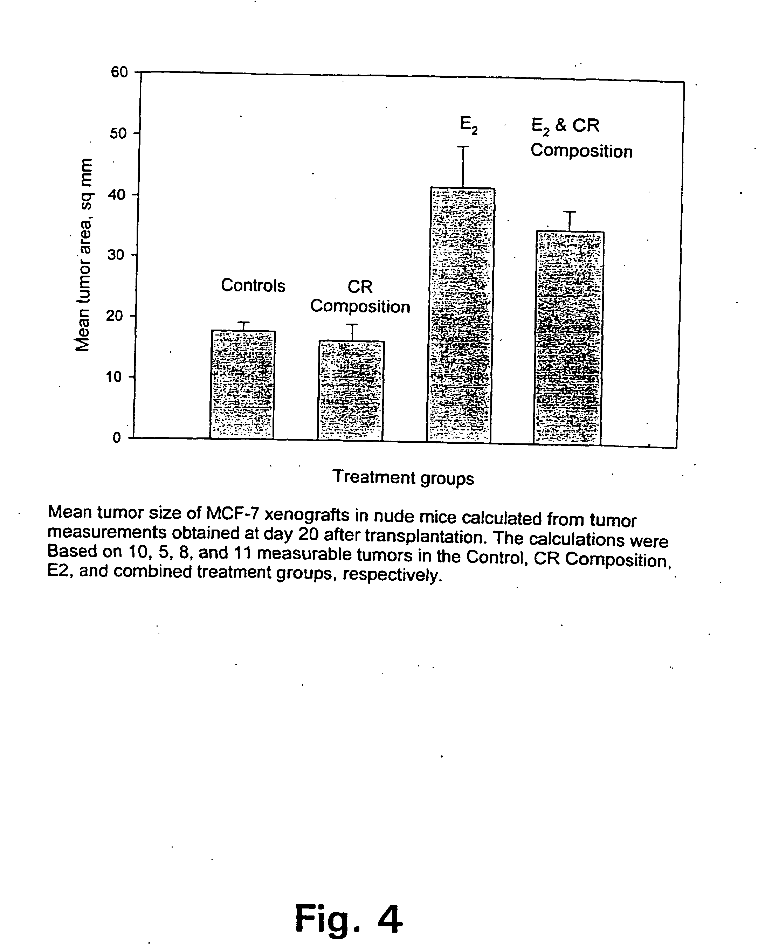

No toxicity was observed.
As expected, the untreated control tumours showed no significant growth. At day 20 after transplantation 10 persisting tumour inocula were evaluable *(Figure 4)*. The SPP-001 treatment did not support the growth of the MCF-7 tumour. The mean tumour area of 5 evaluable tumour inocula at 20 days after transplantation was the same as that of the control group.
The E2 treatment had growth supportive effect on the MCF-7 tumour xenograft. In this group, 8 tumours showed growth, and at day 20 the tumours had reached a mean tumour area of 39 sq mm *(Figure 4)*. The addition of the SPP-001 to E2 did not influence the growth of the tumour. At 20 days after transplantation, the mean tumour area of 11 evaluable tumours in the combined treatment group was the same as that of the E2 group.

Discussion

This study shows that even a 100 fold increase of the SPP-001 did not induce tumours in mice inoculated with hormone receptor positive human breast cancer cells. In contrast, the positive control treatment with E2 has the expected growth supportive effect on the estrogen sensitive and dependent MCF-7 breast cancer xenograft.
In the group where mice received both SPP-001 and estrogen treatment no differences were seen when compared to the estrogen treatment only. This suggests that SPP-001 neither has a potentiating nor an inhibitory (antagonizing) effect on estrogen sensitive breast tumours. The observation further implies that this SPP-001 does not bind to estrogen receptors in MCF-7 cells.

### Example 5: EXPERIMENTAL MODEL FOR TESTING DIFFERENTIAL ESTROGENIC EFFECTS

This example describes an experimental model system for the investigation of drugs or combination of drugs with putative differential estrogenic effects in normal tissues and in breast cancer.

Normal tissue

The possible estrogenic effects on normal tissues are tested using increase in uterine weight as the end point. Alternatively, changes in gonadotropins or vaginal cytology could be used.

The investigation includes an estradiol treatment group which serves as a functional control of the study system.

Vaginal Cytology

Specimen: Smear, collected from vaginal vault or lateral wall, is spread onto pre-labelled glass slides and promptly spray- or wet-fixed.

Method: Papanicolaou staining and microscopy.

Application: Investigation of vaginal bleeding or discharge; follow up smears after previous cervical or endometrial dysplasia or following hysterectomy for malignancy. Cyto-hormonal evaluation is occasionally helpful in assessing hormonal status.

Interpretation: Cytological examination will detect dysplasia, neoplasia, and some infections e.g. infection with Trichomonas vaginalis or Candida albicans.

Animals

Six-eight-week-old female NMRI/Bom™ athymic nude (*nu*/*nu*) mice are used. After acclimatization the mice are ovariectomized in order to avoid influence of endogenous estrogen. The mice are randomised into treatment groups and a group of untreated controls.

The experiments described in this example can be performed with other strains of mice.

Study design

The mice are ovariectomized and randomised at day 0. One group serves as untreated controls (n=5-10), and one group (n=5-10) is given estradiol (E2) from day 1 by insertion of a 0.72 mg subcutaneous slow release E2 pellet (Innovative Research) in the neck of the mice using a trocar.

The last group(s) (n=5-10 in each group) is/are treated with the test composition(s)/drug(s) using relevant dose(s) and schedule(s).

Observations

The animals are observed daily during the experimental period of 8 days. Animals dying before termination of the experiment are excluded from the analysis.

Dosing procedure of test drug(s)

Composition(s) of the drug(s) is/are prepared using appropriate methods for extraction, purification, solubilisation and vehicle systems. The treatment is given by the most convenient route of administration (dermally, orally, intraperitoneally, or intravenously) and with appropriate dosing volume(s).

Dose(s) of the drug(s) is/are selected in relevant dose range(s), and schedule(s) with expected higher efficiency are selected.

Termination of experiment

The experiment is terminated at day 8 after ovariectomy. All animals are sacrificed by cervical dislocation and the mouse uterine weights are determined.

Evaluation

The mouse uterine weight data are used to calculate mean uterine weights of the control, estradiol, and test groups, respectively.

A significant increase in mean uterine weight of the E2 group compared to the controls serves as functional control of the study system.

A significant increase in mean uterine weight of the test drug(s) treatment group(s) are considered evidence of estrogenic effect on normal tissues.

Comparison of the increase in mean uterine weight in the E2 group and test group(s) are used to semiquantitate the estrogenic effect of the investigated drug(s), dose(s), and schedule(s).

Breast cancer

The possible estrogenic effects on breast cancer cells are tested using growth stimulation of an estrogen sensitive human breast cancer xenograft as the end point.

The investigation is performed in two human breast cancer xenografts using nude mice identical to those used for the uterine weight investigation. One tumour, MDA-MB-231 is estrogen and progesterone receptor negative, and therefore estrogen resistant and independent (4). The other tumour, MCF-7 is estrogen and progesterone receptor positive, and estrogen sensitive and dependent (4). The reason for inclusion of the receptor negative breast tumour xenograft in the test system is to identify possible unspecific effects, i.e. non-estrogenic effects which are independent of binding of the drug(s) to estrogen receptors of the tumour cells.

The investigation includes estradiol treatment of the estrogen sensitive MCF-7 tumour xenografts which serves as a functional control of the study system.

Tumour cells

The human mammary cancer cell lines MDA-MB-231 (ATCC HTB-26) and MCF-7 (HTB-22) are used. Near confluent *in vitro* grown cells are harvested using a cell scraper. After centrifugation an aliquot of the cells is stained by Trypan Blue and counted to determine the fraction of viable cells. The cells are resuspended in fresh medium to a final concentration of $10^6$ - $10^7$ viable cells per inoculum (0.1-0.2 ml). The cells are inoculated subcutaneously into both flanks of ovariectomized or intact female nude mice.

Animals

Six-week-old female athymic NMRI/Bom™ nude (*nu/nu*) mice are used. An acclimatization period of one week is allowed in order to exclude animals in poor condition. The experiments described in this example can be performed with other strains of mice.

Ovariectomy is performed under general anaesthesia and using standard procedures following acclimatization of the animals.

In ovariectomized mice inoculation of tumour cells is performed after at least one week to ensure full recovery after the ovariectomy.

After inoculation of cells the animals are randomized into treatment groups according to the study design.

The mice are kept under sterile conditions in laminar air flow clean benches. The mice are allowed sterile water and food pellets *ad libitum.* The room temperature is $25 \pm 2°C$, and the relative humidity $55 \pm 5\%$.

Study design

The growth experiments with the two tumours are performed in separate series using similar study design.

At day 0, the animals are inoculated with tumour cells subcutaneously in both flanks. The mice are then randomized into one group of untreated (vehicle) controls (n=10) and a group (n=10) given E2 from day 1 by insertion of a 0.72 mg subcutaneous slow release E2 pellet (Innovative Research) in the neck of the mice using a trocars. A third group(s) (n=10 in each group) is treated with test drug(s) using relevant dose(s) and schedule(s). The E2 treatment group is only included in the treatment of MCF-7 xenograft.

The dosing procedures are planned as described in the uterine weight section

The animals are observed daily during the experimental period. When the tumours become measurable they are measured in two dimensions three times a week using a sliding gauge. Tumour growth curves are constructed and growth curve parameters are calculated (19).

The experiment is terminated when growing tumours have shown increasing size during at least six consecutive growth recordings. At termination of the experiment, the animals are sacrificed by cervical dislocation.

Evaluation

Using a computer program according to Rygaard et al (19) the tumour measurements are used to construct mean growth curves and to calculate tumour growth curve parameters (19).

Stimulatory effect of E2 on the MCF-7 xenografts serves as functional control of the study system.

A growth supportive effect of the drug(s) in the MCF-7 xenograft indicates an estrogen-like effect mediated through estrogen receptors of the cancer cells. If a growth supportive effect of the drug(s) is also found in the MDA-MB-231 xenograft, this is considered evidence that the effect observed is not mediated through the estrogen receptors of the tumour cells. However, in order to be chosen as a candidate for a substance or composition useful in the method of the invention, the substance or composition should show neither growth supportive effect in the MCF-7 xenograft nor growth supportive effect (which would then be an indirect growth supportive effect) in the MDA-MB-231 xenograft.

Comparison of the growth supportive effect in the E2 group and test group(s) are used to semiquantitate the estrogenic effect of the investigated drug(s), dose(s), and schedule(s) according to Rygaard et al (19).

Similar experiments as those described in this example can be performed using other estrogen receptor-positive and negative human breast cancer cell lines, e.g. T47D, ZR75-1, and MDA-MB-435S (5).

***Example 6: CLINICAL EVALUATION OF THE SAFETY OF SPP-001 IN WOMEN WITH ADVANCED BREAST CANCER***

This example describes a clinical protocol for safety studies of SPP-001 in women with advanced breast cancer.

Patients

Advanced breast cancer patients with measurable disease will be included. The patients should suffer from menopausal symptoms. No chemotherapy or endocrine therapy should be given concomitantly with the SPP-001 treatment. Estrogen receptor (ER) status on the cancer cells should be determined.

Treatment

The SPP-001 should be administered orally twice daily at a corresponding dose of 0.40-0.60 mg SPP-001 /kg. Treatment period should be 3 months.

Study design

Patients will be randomized to either treatment with SPP-001 or placebo. Patients in both treatment groups will be followed until death.

Increased and/or decreased dose(s) and/or administration schedule(s) involving single or multiple dosing of various time intervals can be used.

Evaluation

Measurable lesions (from one to three lesions) should be defined and measured before commencement of SPP-001 treatment. Effect of CR on menopausal symptoms will be registered. Any site effects associated with SPP-001 treatment will be registered. In addition s-FSH and s-LH will be monitored during the treatment period. Furthermore, quantitative PCR or alike can be applied to determine potential effects on estrogen regulated genes such as the progesterone receptor gene and the pS2 gene.

At the end of the experiment measurable lesions will be evaluated and any effect according to WHO criterias will be noted.

Survival curves for each group will be constructed and it will be determined whether SPP-001 has any effects on survival in this patient population.

***Example 7: CLINICAL EVALUATION OF THE SAFETY OF SPP-001 TREATMENT IN WOMEN WITH ADVANCED BREAST CANCER***

This example describes a clinical protocol for safety studies of SPP-001 treatment in women with advanced breast cancer.

Patients

Advanced breast cancer patients who have obtained a complete or partial response upon conventional antineoplastic therapy will be included. The patients should suffer from menopausal symptoms. No chemotherapy or endocrine therapy should be given concomitantly with the SPP-001 treatment. ER status on the cancer cells should be determined.

Treatment

The SPP-001 should be administered orally twice daily at a corresponding dose of 0.40-0.60 mg SPP-001 /kg. Treatment period should be 3 months.

Study design

Patients will be randomized to either treatment with SPP-001 or placebo and followed until death.
Dose(s) and schedule(s) can be subjected to changes.

Evaluation

Time of disease progression using available WHO criterias will be used. Effect of SPP-001 on menopausal symptoms and gonadotropins will be registered. Any side effects associated with SPP-001 composition treatment will be registered.
Time to progression in each treatment group will be determined and compared between groups in order to determine whether SPP-001 has any effect on cancer progression in this patient population.

*Example 8: CLINICAL EVALUATION OF THE SAFETY OF SPP-001 IN WOMEN WHO HAVE HAD A PRIOR BREAST CANCER*

This example describes a clinical protocol for safety studies of SPP-001 in women with a prior diagnosis of breast cancer and with no indication of recurrence.

Patients

Women with a prior diagnosis of breast cancer who are suffering from menopausal symptoms will be included. The patients must have no sign of recurrent disease. No antineoplastic treatment should be given concomitantly with the SPP-001 treatment. ER status on the cancer cells should be determined.

Treatment

The SPP-001 should be administered orally twice daily at a corresponding dose of 0.40-0.60 mg SPP-001 /kg. Treatment period should be 1 year.

Study design

Patients should be randomized to either treatment with SPP-001 or placebo. Patients in both groups will be followed for 5 years.
Dose(s) and schedule(s) can be subjected to changes.

Evaluation

Time to recurrence of disease will be determined. Effect of SPP-001 on menopausal symptoms and gonadotropins will be registered. Any side-effects associated SPP-001 treatment will be registered.
Univariate recurrence-free survival and overall survival curves will be constructed for both treatment groups and compared in order to establish whether the treatment has any effect on these two parameters.

**Example 9:** *EFFECT OF SPP-001 ON ESTROGEN DEFICIENCY CONDITIONS OTHER THAN MENOPAUSAL SYMPTOMS IN PATIENTS WITH CURRENT BREAST CANCER, WITH A PRIOR DIAGNOSIS OF BREAST CANCER, OR WITH AN INCREASED RISK OF DEVELOPING BREAST CANCER*

This example describes SPP-001 treatment of estrogen deficiency conditions including dermatological disorders such as ageing of the skin, dryness of mucous membranes (e.g. vaginal and intestine), brain related disease such as Alzheimer's including other types of dementia, bone and joint related disease such as osteoporosis, osteochondrosis, osteoarthritis, rheumatoid arthritis, healing of bone fractures, and reduce in skeletal fractures and disease such as hyperlipidaemia, hypercholesterolaemia, arteriosclerosis, etc. in the subgroup of women who are currently suffering from breast cancer, who have a prior history of breast cancer, or women with an increased risk of developing breast cancer.

Patients

Women belonging to one of the above mentioned breast cancer related groups and suffering from estrogen deficiency, will be candidates for the treatment. Diseases related to estrogen deficiency includes among others those mentioned above.
In addition, women belonging to one of the above mentioned three breast cancer related groups and suffering from diseases which can be cured or relieved by estrogen replacement therapy, can be included in this treatment study.

Treatment

SPP-001 should be administered twice daily at a corresponding dose of 0.40-0.60 SPP-001 /kg.
Dose(s) and schedule(s) can be subjected to changes.

**Example 10:** *CLINICAL EVALUATION OF PHYSIOLOGICAL ESTROGENIC EFFECT OF SPP-001 IN MENOPAUSAL WOMEN*

This example describes methods to determine physiological estrogenic effects of SPP-001 and similar compositions in the human female suffering from estrogen deficiencies.

Patients

Women with high FSH and/or elevated LH as a reaction to estrogen deficiency

Material and design

Serum levels of FSH and LH will be determined pre-treatment and at 1 month and 2 months of treatment. Standard commercial assays will be used. Vaginal smear cytology will be performed before and after treatment for 60 days. Bone mineral density will be determined pre-treatment and following two months of treatment. Standard DXA assay will be used.

**Example 11:** *SKIN APPLICATIONS OF SPP-001 OR ALIKE FOR CONDITIONS WHICH COULD BE ALLEVIATED OR CORRECTED BY ESTROGENS*

Material

Humans with as for example wrinkles, dry skin and other estrogen deficiency related conditions.

Treatment

Individuals with the conditions in question are treated with daily SPP-001 or similar with relieve of conditions as endpoint. Typical treatment period is 1-2 months. Route of administration is preferably direct skin application but can also be oral or parenteral.

### Example 12: VAGINAL APPLICATION OF SPP-001 TO ALLEVIATE SYMPTOMS OF VAGINAL DRYNESS AND ESTROGEN DEFICIENCY RELATED CONDITIONS

Materials

Women with symptoms of vaginal estrogen deficiency such as dryness and dyspareuni.

Treatment

An appropriate dose of SPP-001 is given to alleviate the vaginal symptoms by direct application to the mucous membrane or by oral or parenteral administration. Used when needed.

### Example 13: APPLICATIONS OF SPP-001 OR ALIKE FOR CORONARY HEART APPLICATION (CHD) CONDITIONS WHICH COULD BE ALLEVIATED OR CORRECTED BY ESTROGENS

Estrogens have been generally regarded as cardiac protective in the female population. SPP-001 and alike can be used because of their estrogen beneficial activity, but without its unwanted negative issues, for treatment and alleviation of symptoms of arterial and venous diseases, including heart and brain.

Material

Humans with as for examples arteriosclerosis.

Treatment

individuals with the conditions in question are treated with daily SPP-001 or similar with a lowering of cholesterol or death in cardiac disease as endpoint. Typical treatment period is from 2 months to 5 years. Route of administration is preferably orally, but can also be oral or parenteral.

## REFERENCES

1    Beuscher, N. Cimicifuga racemosa L. - Die Traubensilberkerze. Z.Phytotherapei, 16: 301-310, 1995.

2    Brünner, N., Bastert, G.B.A., Poulsen, H.S., Spang-Thomsen, M., Engelholm, S.A., Vindeløv, L., Nielsen, A., Tommerup, N., and Elling, F. Characterization of the T61 human breast carcinoma established in nude mice. Eur.J.Cancer Clin.Oncol., 21: 833-843, 1985.

3    Brünner, N., Bronzert, D., Vindeløv, L.L., Rygaard, K., Spang-Thomsen, M., and Lippman, M.E. Effect on growth and cell cycle kinetics of estradiol and tamoxifen on MCF-7 human breast cancer cells grown in vitro and in Nude mice. Cancer Res., 49: 1515-1520, 1989.

4    Brünner, N., Moser, C., Clarke, R., and Cullen, K. IGF-I and IGF-II expression in human breast cancer xenografts: relationship to hormone independence. Breast Cancer Res.Treat., 22: 39-45, 1992.

5    Brünner, N., Osborne, C.K., and Spang-Thomsen, M. Endocrine therapy of human breast cancer grown in nude mice. Breast Cancer Res.Treat., 10: 229-242, 1987.

6    Brünner, N., Spang-Thomsen, M., Poulsen, H.S., Engelholm, S.A., Nielsen, A., and Vindeløv, L. Endocrine sensitivity of the receptor-positive T61 human breast carcinoma serially grown in nude mice. Int.J.Cancer, 35: 59-64, 1985.

7    Brünner, N., Spang-Thomsen, M., Poulsen, H.S., Vindeløv, L.L., and Engelholm, S.A. Characterization of the T60 human breast carcinoma grown in nude mice. In: J. Rygaard, N. Brünner, N. Græm and M. Spang-Thomsen (eds.), Immune-Deficient Animals in Biomedical Research, pp. 234-241, Basel: Karger. 1987.

8    Brünner, N., Spang-Thomsen, M., Vindeløv, L., and Nielsen, A. Effect of 17□-oestradiol on growth curves and flow cytometric DNA distribution of two human breast carcinomas grown in nude mice. Br.J.Cancer, 47: 641-647, 1983.

9    Brünner, N., Spang-Thomsen, M., Vindeløv, L., Nielsen, A., Engelholm, S.A., and Svenstrup, B. Dose-dependent effect of 17 β-estradiol determined by growth curves and flow cytometric DNA analysis of a human breast carcinoma (T61) grown in nude mice. Exp.Cell Biol., 53: 220-232, 1985.

10   Brünner, N., Spang-Thomsen, M., Vindeløv, L., Wolff, J., and Engelholm, S.A. Effect of tamoxifen on the receptor-positive T61 and the receptor-negative T60 human breast carcinomas grown in nude mice. Eur.J.Cancer Clin.

Oncol., 21: 1349-1354, 1985.

11 Brünner, N., Svenstrup, B., Spang-Thomsen, M., Bennet, P., Nielsen, A., and Nielsen, J. Serum steroid levels in intact and endocrine ablated BALB/c nude mice and their intact littermates. J.Steroid Biochem., 25: 429-432, 1986.

12 Düker, E.-M., Kopanski, L., Jarry, H., and Wuttke, W. Effects of extracts from Cimicifuga racemosa on godnadotropin release in menopausal women. Planta Med., 57: 420-424, 1991.

13 Harnischfeger, G. and Stolze, H. Traubensilberkerze. Notabene Medici, 10: 446-450, 1980.

14 Jarry, H., Gorkow, C., and Wuttke, W. Treatment of menopausal symptoms with extracts of Cimicifuga Racemosa: In vivo and in vitro evidence for estrogenic activity. Unknown, 1999.

15 Liske, E. and Düker, E. Cimicifuga racemosa in Klinik und Forschung. Ars Medici, 7: 426-430, 1993.

16 Martin, P.M., Horwitz, K.B., Ryan, D.S., and McGuire, W.L. Phytoestrogen interaction with estrogen receptors in human breast cancer cells. Endocrinology, 103: 1860-1867, 1978.

17 McGuire, W.L. Current status of estrogen receptors in human breast cancer. Cancer, 36: 638-644, 1975.

18 McGuire, W.L. Steroid receptors in human breast cancer. Cancer Res., 38: 4289-4291, 1978.

19 Rygaard, K. and Spang-Thomsen, M. Quantitation and Gompertzian analysis of tumour growth. Breast Cancer Res.Treat., 46: 303-312, 1997.

20 Warnecke, G. Beeinflussung klimakterischer Beschwerden durch ein Phytotherapeutikum. Medwelt., 36: 871-874, 1985.

21 Vorberg, G. Therapie klimakterischer Beschwerden. Z.Allg.Med., 60: 626-629, 1984.

22 Gottardis MM., Wagner RJ., Borden EC., and Jordan VC. Differential ability of antiestrogens to stimulate breast cancer cell (MCF-7) growth in vivo and in vitro. Cancer Res 1989 49(17):4765-69

**Claims**

1. Use of a composition comprising substances contained in *Cimicifuga Racemosa* extract, which induces a physiological estrogen-like effect without interacting with breast cancer cells, for the preparation of a medicament for the treatment of estrogen deficiency symptoms or diseases of a mammal suffering from or having a high risk of developing breast cancer.

2. Use according to claim 1, wherein the composition induces a physiological estrogen-like effect without stimulating breast cancer cells.

3. Use according to any of the preceding claims, wherein the physiological estrogen-like effect is uterine growth as determined by an increase in uterine weight compared to controls after administration of the composition to ovariectomized female athymic nude mice.

4. Use according to any of the preceding claims, wherein the physiological estrogen-like effect is uterine growth as determined by an increase in mean uterine weight compared to controls of at least 0.10 g after administration of the composition to ovariectomized NMRI female athymic nude mice for 8 days.

5. Use according to any of the preceding claims, wherein the physiological estrogen-like effect is a change in gonadotropins (FSH and/or LH) as determined by available validated radioimmuno assay techniques.

6. Use according to any of the preceding claims, wherein the physiological estrogen-like effect is a change in cytology of the vaginal cells as determined by cytological counts.

7. Use according to any of the preceding claims, wherein the composition does not interact, in particular stimulate, cancer cells that are estrogen receptor-negative.

8. Use according to any of the preceding claims, wherein the lack of stimulation of breast cancer cells is determined by no effect of the composition compared to a control on growth of the estrogen and progesterone receptor negative MDA-MB-231 (ATCC HTB-26) human breast cancer cell line inoculated into six-week-old female athymic nude mice determined when control tumours show increasing size during at least six consecutive growth recordings.

9. Use according to any of the preceding claims, wherein the composition does not interact, in particular stimulate, cancer cells that are estrogen receptor-positive.

**10.** Use according to any of the preceding claims, wherein the composition is one which has substantially no agonizing and substantially no antagonizing effect on the effect of estrogen such as estradiol on breast cancer cells, even where the breast cancer cells are documented as being estrogen receptor-positive.

**11.** Use according to any of the preceding claims, wherein the composition is one which substantially does not bind to estrogen receptors of cancer cells.

**12.** Use according to any of the preceding claims, wherein the lack of stimulation of breast cancer cells is determined by no effect of the composition compared to a control on growth of the estrogen dependent and estrogen receptor-positive MCF-7 (ATCC HTB-22) human breast cancer cell line inoculated into six-week-old female athymic-nude mice determined when control tumours show increasing size during at least six consecutive growth recordings.

**13.** Use according to any of the preceding claims, wherein the lack of stimulation of breast cancer cells is determined by no effect of the composition when given in combination with estradiol compared to a control on growth of the estrogen dependent and estrogen receptor-positive MCF-7 (ATCC HTB-22) human breast cancer cell line inoculated into six-week-old female athymic nude mice determined when control tumours show increasing size during at least six consecutive growth recordings.

**14.** Use according to any of claims 15 or 16, wherein the lack of stimulation of breast cancer cells is determined by no effect of the composition, even where the composition is administered in a dose which is 10 or even 100 times higher than a dose which in the same strain of nude mice induces a maximum uterus weight increase.

**15.** Use according to any of the preceding claims for the treatment of estrogen deficiency symptoms or diseases of women having breast cancer, having a high risk of recurrent breast cancer, or having a risk (such as high risk) of developing breast cancer.

**16.** Use according to any of the preceding claims, wherein the estrogen deficiency conditioned symptoms are menopausal symptoms.

**17.** Use according to any of the preceding claims for the treatment of dermatological disorders such as ageing of the skin, wrinkles, dry skin and other estrogen deficiency related dermatological disorders of women having breast cancer, having a high risk of recurrent breast cancer, or having a risk (such as high risk) of developing breast cancer.

**18.** Use according to any of the preceding claims for the treatment of dryness of mucous membranes, e.g. vaginal and intestine, of women having breast cancer, having a high risk of recurrent breast cancer, or having a risk (such as high risk) of developing breast cancer.

**19.** Use according to any of the preceding claims for the treatment of brain related disease such as Alzheimer's including other types of dementia of women having breast cancer, having a high risk of recurrent breast cancer, or having a risk (such as high risk) of developing breast cancer.

**20.** Use according to any of the preceding claims for the treatment of bone and joint related diseases such as osteoporosis, osteochondrosis, osteoarthritis, rheumatoid arthritis, healing of bone fractures, and reduction in skeletal fractures of women having breast cancer, having a high risk of recurrent breast cancer, or having a risk (such as high risk) of developing breast cancer.

**21.** Use according to any of the preceding claims for the treatment of vaginal estrogen deficiency such as vaginal dryness and dyspareuni of women having breast cancer, having a high risk of recurrent breast cancer, or having a risk (such as high risk) of developing breast cancer.

**22.** Use according to any of the preceding claims for the treatment of coronary heart diseases such as arteriosclerosis of women having breast cancer, having a high risk of recurrent breast cancer, or having a risk (such as high risk) of developing breast cancer.

**23.** Use according to any of the preceding claims for the treatment of estrogen deficiency-conditioned diseases such as hyperlipidaemia and hypercholesterolaemia of women having breast cancer, having a high risk of recurrent breast cancer, or having a risk (such as high risk) of developing breast cancer.

**24.** Use according to any of the preceding claims, wherein the composition is *Cimicifuga Racemosa* extract.

**25.** Use according to any of the preceding claims, wherein the composition is a composition comprising *Cimicifuga Racemosa* plant parts.

**26.** Use according to any of the preceding claims, wherein the composition is a composition comprising SPP-001.

**27.** Use according to any of the preceding claims, wherein the composition is a composition containing one or more chemical compounds contained in *Cimicifuga Racemosa* extract.

**28.** Use according to any of the preceding claims, wherein the composition is combined with a drug which has a selective estrogen receptor modulating (SERM) actitivty.

**Patentansprüche**

**1.** Verwendung einer Zusammensetzung, umfassend Stoffe, die in einem *Cimicifuga Racemosa*-Extrakt enthalten sind, die eine physiologische, Östrogen-ähnliche Wirkung hervorruft, ohne mit Brustkrebszellen zu interagieren, für die Herstellung eines Medikaments zur Behandlung von Östrogenmangelsymptomen oder Krankheiten eines Säugers, der an Brustkrebs leidet oder ein hohes Risiko hat, Brustkrebs zu entwickeln.

**2.** Verwendung nach Anspruch 1, wobei die Zusammensetzung eine physiologische, Östrogen-ähnliche Wirkung hervorruft, ohne Brustkrebszellen zu stimulieren.

**3.** Verwendung nach einem der vorangehenden Ansprüche, wobei die physiologische, Östrogen-ähnliche Wirkung Gebärmutterwachstum ist, bestimmt durch eine Zunahme des Gebärmuttergewichts, im Vergleich zu Kontrollen, nach Verabreichung der Zusammensetzung an ovariektomierte weibliche athymische Nacktmäuse.

**4.** Verwendung nach einem der vorangehenden Ansprüche, wobei die physiologische, Östrogen-ähnliche Wirkung Gebärmutterwachstum ist, bestimmt durch eine Zunahme des mittleren Gebärmuttergewichts um zumindest 0,10 g, im Vergleich zu Kontrollen, nach Verabreichung der Zusammensetzung an ovariektomierte weibliche athymische NMRI-Nacktmäuse für 8 Tage.

**5.** Verwendung nach einem der vorangehenden Ansprüche, wobei die physiologische, Östrogen-ähnliche Wirkung eine Änderung der Gonadotropine (FSH und/oder LH) ist, bestimmt durch verfügbare validierte Radioimmunassay-Techniken.

**6.** Verwendung nach einem der vorangehenden Ansprüche, wobei die physiologische, Östrogen-ähnliche Wirkung eine Änderung der Cytologie der Vaginalzellen ist, bestimmt durch cytologische Zählungen.

**7.** Verwendung nach einem der vorangehenden Ansprüche, wobei die Zusammensetzung nicht mit Krebszellen interagiert, insbesondere diese nicht stimuliert, die Östrogenrezeptor-negativ sind.

**8.** Verwendung nach einem der vorangehenden Ansprüche, wobei die fehlende Stimulation der Brustkrebszellen bestimmt wird durch die fehlende Wirkung der Zusammensetzung, im Vergleich zu einer Kontrolle, auf das Wachstum der Ostrogen-und Progesteronrezeptor-negativen menschlichen Brustkrebzelllinie MDA-MB-231 (ATCC HTB-26), die in sechs Wochen alte weibliche athymische Nacktmäuse inokuliert wurde, was bestimmt wird, wenn Kontrolltumoren eine zunehmende Größe während zumindest sechs aufeinanderfolgender Wachstumsregistrierungen zeigen.

**9.** Verwendung nach einem der vorangehenden Ansprüche, wobei die Zusammensetzung nicht mit Krebszellen interagiert, insbesondere diese nicht stimuliert, die Östrogenrezeptor-positiv sind.

**10.** Verwendung nach einem der vorangehenden Ansprüche, wobei die Zusammensetzung eine solche ist, die im Wesentlichen keine agonistische und im Wesentlichen keine antagonistische Wirkung auf die Wirkung von Östrogen, wie z.B. Östradiol, auf Brustkrebszellen hat, sogar bei Brustkrebszellen, die als Östrogenrezeptor-positiv dokumentiert sind.

**11.** Verwendung nach einem der vorangehenden Ansprüche, wobei die Zusammensetzung eine solche ist, die im Wesentlichen nicht an Östrogenrezeptoren von Krebszellen bindet.

**12.** Verwendung nach einem der vorangehenden Ansprüche, wobei die fehlende Stimulation der Brustkrebszellen bestimmt wird durch die fehlende Wirkung der Zusammensetzung, im Vergleich zu einer Kontrolle, auf das Wachstum der Östrogen-abhängigen und Östrogenrezeptor-positiven menschlichen Brustkrebszelllinie MCF-7 (ATCC HTB-22), die in sechs Wochen alte weibliche athymische Nacktmäuse inokuliert wurde, was bestimmt wird, wenn Kontrolltumoren eine zunehmende Größe während zumindest sechs aufeinanderfolgender Wachstumsregistrierungen zeigen.

**13.** Verwendung nach einem der vorangehenden Ansprüche, wobei die fehlende Stimulation der Brustkrebszellen bestimmt wird durch die fehlende Wirkung der Zusammensetzung, nach Verabreichung in Kombination mit Östradial im Vergleich zu einer Kontrolle, auf das Wachstum der Östrogen-abhängigen und Östrogenrezeptor-positiven menschlichen Brustkrebszelllinie MCF-7 (ATCC HTB-22), die in sechs Wochen alte weibliche athymische Nacktmäuse inokuliert wurde, was bestimmt wird, wenn Kontrolltumoren eine zunehmende Größe während zumindest sechs aufeinanderfolgender Wachstumsregistrierungen zeigen.

**14.** Verwendung nach einem der Ansprüche 15 oder 16, wobei die fehlende Stimulation der Brustkrebszellen durch fehlende Wirkung der Zusammensetzung bestimmt wird, sogar wenn die Zusammensetzung in einer Dosis verabreicht wird, die 10 oder sogar 100 mal höher ist als eine Dosis, die in der selben Nacktmauslinie eine maximale Gebärmuttergewichtszunahme bewirkt.

**15.** Verwendung nach einem der vorangehenden Ansprüche zur Behandlung von Östrogenmangelsymptomen oder Krankheiten von Frauen, die Brustkrebs haben, die ein hohes Risiko von rezidivierendem Brustkrebs haben oder die ein Risiko haben (beispielsweise ein hohes Risiko), Brustkrebs zu entwickeln.

**16.** Verwendung nach einem der vorangehenden Ansprüche, wobei die Östrogenmangel-bedingten Symptome Menopausensymptome sind.

**17.** Verwendung nach einem der vorangehenden Ansprüche zur Behandlung von dermatologischen Störungen, wie Alterung der Haut, Falten, trockene Haut und anderer, mit Östrogenmangel verwandter dermatologischer Störungen von Frauen, die Brustkrebs haben, die ein hohes Risiko von rezidivierendem Brustkrebs haben oder die ein Risiko haben (beispielsweise ein hohes Risiko), Brustkrebs zu entwickeln.

**18.** Verwendung nach einem der vorangehenden Ansprüche zur Behandlung der Trockenheit von mukösen Membranen, z.B. der Vagina und des Darms, von Frauen, die Brustkrebs haben, ein hohes Risiko von rezidivierendem Brustkrebs oder ein Risiko (beispielsweise als hohes Risiko), Brustkrebs zu entwickeln.

**19.** Verwendung nach einem der vorangehenden Ansprüche zur Behandlung von auf das Gehirn bezogener Krankheiten, wie Alzheimer, einschließlich anderer Demenzarten, von Frauen, die Brustkrebs haben, die ein hohes Risiko von rezidivierendem Brustkrebs haben oder die ein Risiko haben (beispielsweise ein hohes Risiko), Brustkrebs zu entwickeln.

**20.** Verwendung nach einem der vorangehenden Ansprüche zur Behandlung von auf Knochen und Gelenke bezogener Krankheiten, wie Osteoporose, Osteochondrose, Osteoarthritis, rheumatoider Arthritis, Heilung von Knochenfrakturen und Verminderung von Skelettfrakturen bei Frauen, die Brustkrebs haben, die ein hohes Risiko von rezidivierendem Brustkrebs haben oder die ein Risiko haben (beispielsweise ein hohes Risiko), Brustkrebs zu entwickeln.

**21.** Verwendung nach einem der vorangehenden Ansprüche zur Behandlung von vaginalem Östrogenmangel, wie vaginaler Trockenheit und Dyspareunie, bei Frauen, die Brustkrebs haben, die ein hohes Risiko von rezidivierendem Brustkrebs haben oder die ein Risiko haben (beispielsweise ein hohes Risiko), Brustkrebs zu entwickeln.

**22.** Verwendung nach einem der vorangehenden Ansprüche zur Behandlung koronarer Herzkrankheiten, wie Arteriosklerose von Frauen, die Brustkrebs haben, die ein hohes Risiko von rezidivierendem Brustkrebs haben oder die ein Risiko haben (beispielsweise ein hohes Risiko), Brustkrebs zu entwickeln.

**23.** Verwendung nach einem der vorangehenden Ansprüche zur Behandlung von Krankheiten, die durch Östrogenmangel bedingt sind, wie Hyperlipidämie und Hypercholesterinämie von Frauen, die Brustkrebs haben, die ein hohes Risiko von rezidivierendem Brustkrebs haben oder die ein Risiko haben (beispielsweise ein hohes Risiko), Brustkrebs zu entwickeln.

**24.** Verwendung nach einem der vorangehenden Ansprüche, wobei die Zusammensetzung ein *Cimicifuga Racemosa*-Extrakt ist.

**25.** Verwendung nach einem der vorangehenden Ansprüche, wobei die Zusammensetzung eine Zusammensetzung ist, die *Cimicifuga Racemosa*-Pflanzenteile umfasst.

**26.** Verwendung nach einem der vorangehenden Ansprüche, wobei die Zusammensetzung eine Zusammensetzung ist, die SPP-001 umfasst.

**27.** Verwendung nach einem der vorangehenden Ansprüche, wobei die Zusammensetzung eine Zusammensetzung ist, die einen oder mehrere chemische Stoffe enthält, die in einem *Cimicifuga Racemosa*-Extrakt enthalten sind.

**28.** Verwendung nach einem der vorangehenden Ansprüche, wobei die Zusammensetzung mit einem Wirkstoff kombiniert wird, der eine selektive Östrogenrezeptor-modulierende (SERM) Aktivität hat.

**Revendications**

**1.** Utilisation d'une composition comprenant des substances contenues dans un extrait de *Cimicifuga Racemosa,* qui induit un effet de type oestrogène physiologique sans interagir avec les cellules du cancer du sein, pour la préparation d'un médicament pour le traitement de symptômes ou de maladies de déficience en oestrogènes d'un mammifère souffrant ou présentant un risque élevé de développer un cancer du sein.

**2.** Utilisation selon la revendication 1, dans laquelle la composition induit un effet de type oestrogène physiologique sans stimuler les cellules du cancer du sein.

**3.** Utilisation selon l'une quelconque des revendications précédentes, dans laquelle l'effet de type oestrogène physiologique est la croissance utérine, telle que déterminée par une augmentation du poids utérin, en comparaison avec des contrôles, après l'administration de la composition à des souris nude thymoprives femelles ayant subi une ovariectomie.

**4.** Utilisation selon l'une quelconque des revendications précédentes, dans laquelle l'effet de type oestrogène physiologique est la croissance utérine telle que déterminée par une augmentation du poids utérin moyen en comparaison avec des contrôles d'au moins 0,10 g, après l'administration de la composition à des souris nude thymoprives femelles NMRI ayant subi une ovariectomie, pendant 8 jours.

**5.** Utilisation selon l'une quelconque des revendications précédentes, dans laquelle l'effet de type oestrogène physiologique est une modification des gonadotrophines (FSH et/ou LH) telle que déterminée par des techniques de dosages radioimmunologiques validées disponibles.

**6.** Utilisation selon l'une quelconque des revendications précédentes, dans laquelle l'effet de type oestrogène physiologique est une modification dans la cytologie des cellules vaginales telles que déterminés par des comptages cytologiques.

**7.** Utilisation selon l'une quelconque des revendications précédentes, dans laquelle la composition n'interagit pas, en particulier ne stimule pas, les cellules cancéreuses qui sont négatives aux récepteurs des oestrogènes.

**8.** Utilisation selon l'une quelconque des revendications précédentes, dans laquelle le manque de stimulation des cellules du cancer du sein est déterminée par l'absence d'effet de la composition en comparaison avec un contrôle sur la croissance de la lignée de cellules de cancer du sein humaine MDA-MB-231 (ATCC HTB-26) négatives aux récepteurs des oestrogènes et de la progestérone inoculée dans des souris nude thymoprives femelles âgées de six semaines déterminées quand les tumeurs de contrôle présentent une augmentation de taille pendant au moins six enregistrements de croissance consécutifs.

**9.** Utilisation selon l'une quelconque des revendications précédentes, dans laquelle la composition n'interagit pas, en particulier ne stimule pas, les cellules cancéreuses qui sont positives aux récepteurs des oestrogènes.

**10.** Utilisation selon l'une quelconque des revendications précédentes, dans laquelle la composition en est une qui n'a substantiellement aucun effet agoniste et substantiellement aucun effet antagoniste sur l'effet des oestrogènes tels que l'oestradiol sur les cellules du cancer du sein, même quand les cellules du cancer du sein sont documentées comme étant positives aux récepteurs des oestrogènes.

**11.** Utilisation selon l'une quelconque des revendications précédentes, dans laquelle la composition en est une qui ne se fixe substantiellement pas aux récepteurs des oestrogènes des cellules cancéreuses.

**12.** Utilisation selon l'une quelconque des revendications précédentes, dans laquelle le manque de stimulation des cellules du cancer du sein est déterminé par l'absence d'effet de la composition en comparaison avec un contrôle de la croissance de la lignée des cellules de cancer du sein humain MCF-7 (ATCC HTB-22) positives aux récepteurs des oestrogènes et dépendantes des oestrogènes, inoculée dans des souris nude thymoprives femelles âgées de six semaines, déterminée quand les tumeurs de contrôle présentent une augmentation de la taille pendant au moins six enregistrements consécutifs.

**13.** Utilisation selon l'une quelconque des revendications précédentes, dans laquelle le manque de stimulation des cellules du cancer du sein est déterminé par l'absence d'effet de la composition quand elle est donnée en combinaison avec l'oestradiol en comparaison avec un contrôle de la croissance de la lignée des cellules de cancer du sein humaine MCF-7 (ATCC HTB-22) positives aux récepteurs des oestrogènes et dépendantes des oestrogènes, inoculée dans des souris nude thymoprives femelles âgées de six semaines, déterminée quand les tumeurs de contrôle présentent une augmentation de la taille pendant au moins six enregistrements de croissance consécutifs.

**14.** Utilisation selon l'une quelconque des revendications 15 ou 16, dans laquelle le manque de stimulation des cellules du cancer du sein est déterminé par l'absence d'effet de la composition, même quand la composition est administrée à une posologie qui est 10 ou même 100 fois supérieure à une posologie donnant, dans la même souche des cellules souris nude, une augmentation du poids de l'utérus maximum.

**15.** Utilisation selon l'une quelconque des revendications précédentes, pour le traitement de symptômes ou de maladies de déficience en oestrogènes de femmes ayant un cancer du sein, ayant un risque élevé de cancer du sein récurrent
ou ayant un risque (tel qu'un risque élevé) de développer un cancer du sein.

**16.** Utilisation selon l'une quelconque des revendications précédente, dans laquelle les symptômes conditionnés par la déficience en oestrogènes sont des symptômes de la ménopause.

**17.** Utilisation selon l'une quelconque des revendications précédentes, pour le traitement de troubles dermatologiques tels que le vieillissement de la peau, les rides, la sécheresse cutanée et d'autres troubles dermatologique liés à la déficience en oestrogènes des femmes ayant un cancer du sein, ayant un risque élevé de cancer du sein récurrent ou ayant un risque (tel qu'un risque élevé) de développer un cancer du sein.

**18.** Utilisation selon l'une quelconque des revendications précédentes, pour le traitement de la sécheresse des membranes muqueuses, par exemple vaginales et intestinales, de femmes ayant un cancer du sein, ayant un risque élevé de cancer du sein récurrent ou ayant un risque (tel qu'un risque élevé) de développer un cancer du sein.

**19.** Utilisation selon l'une quelconque des revendications précédentes, pour le traitement de maladies liées au cerveau telles que la maladie d'Alzheimer y compris d'autres types de démence de femmes ayant un cancer du sein, ayant un risque élevé de cancer du sein récurrent ou ayant un risque (tel qu'un risque élevé) de développer un cancer du sein.

**20.** Utilisation selon l'une quelconque des revendications précédentes, pour le traitement de maladies associées aux os et aux articulations telles que l'ostéoporose, l'ostéochondrose, l'arthrose, la polyarthrite rhumatoide, la guérison de fractures osseuses et la réduction des fractures du squelette, de femmes ayant un cancer du sein, ayant un risque élevé de cancer du sein récurrent ou ayant un risque (tel qu'un risque élevé) de développer un cancer du sein.

**21.** Utilisation selon l'une quelconque des revendications précédentes, pour le traitement d'une déficience vaginale en oestrogènes, telle que la sécheresse vaginale et la dyspareunie, de femmes ayant un cancer du sein, ayant un risque élevé de cancer du sein récurrent ou ayant un risque (tel qu'un risque élevé) de développer un cancer du sein.

**22.** Utilisation selon l'une quelconque des revendications précédentes, pour le traitement de maladies cardiaques coronaires, telles que l'artériosclérose, de femmes ayant un cancer du sein, ayant un risque élevé de cancer du sein récurrent ou ayant un risque (tel qu'un risque élevé) de développer un cancer du sein.

**23.** Utilisation selon l'une quelconque des revendications précédentes, pour le traitement de maladies conditionnées par une déficience en oestrogènes, telles que l'hyperlipidémie et l'hypercholestérolémie, de femmes ayant un cancer du sein, ayant un risque élevé de cancer du sein récurrent ou ayant un risque (tel qu'un risque élevé) de développer un cancer du sein.

**24.** Utilisation selon l'une quelconque des revendications précédentes, dans laquelle la composition est un extrait de *Cimicifuga Racemosa.*

**25.** Utilisation selon l'une quelconque des revendications précédentes, dans laquelle la composition est une composition comprenant des parties de la plante *Cimicifuga Racemosa.*

**26.** Utilisation selon l'une quelconque des revendications précédentes, dans laquelle la composition est une composition comprenant SPP-001.

**27.** Utilisation selon l'une quelconque des revendications précédentes, dans laquelle la composition est une composition comprenant un ou plusieurs composés chimiques contenus dans un extrait de *Cimicifuga Racemosa.*

**28.** Utilisation selon l'une quelconque des revendications précédentes, dans laquelle la composition est combinée à un médicament qui a une activité de modulation du récepteur des oestrogènes sélective (SERM).

Effect of estradiol ($E_2$) and CR Composition on mouse uterine weight.

# Fig. 1

## MDA-MD-231 BREAST CARCINOMA

Mean tumor area growth curves of human MDA-MB-231 breast carcinoma xenografts. The mice were untreated controls or treated with CR Composition.

# Fig. 2

## MCF-7 BREAST CARCINOMA

Mean tumor area growth curves of human MCF-7 breast carcinoma xenografts. The mice were treated with $E_2$ or CR Composition. The lack of tumor growth in CR Composition treated mice are indicated in the figure.

# Fig. 3

Mean tumor size of MCF-7 xenografts in nude mice calculated from tumor measurements obtained at day 20 after transplantation. The calculations were Based on 10, 5, 8, and 11 measurable tumors in the Control, CR Composition, E2, and combined treatment groups, respectively.

# Fig. 4